# EUROPÄISCHE PATENTSCHRIFT

(11) **EP 0 568 915 B1**
(45) Veröffentlichungstag und Bekanntmachung des Hinweises auf die Patenterteilung: **20.05.1998**
(21) Anmeldenummer: 93106877.9
(22) Anmeldetag: 28.04.1993
(51) Int. Cl.: C07C 309/71, C07C 309/76, C07C 303/42, G03F 7/023

(54) **Verfahren zur Phlegmatisierung von 1,2-Naphthochinon-2-diazidsulfonsäureestern**
Process for the desensitization of 1,2-naphthoquinone-2-diazide-sulfonic acid esters
Procédé pour la désensibilisation d'esters sulfonique de naphthoquinone 1,2-diazide-2

(30) Priorität: 06.05.1992 DE 4219544
(43) Veröffentlichungstag der Anmeldung: 10.11.1993
(73) Patentinhaber: Agfa-Gevaert AG, 51373 Leverkusen (DE)
(72) Erfinder: Erdmann, Fritz, Dr., W-6228 Eltville-Martinsthal (DE); Kinkel, Tonio, Dr., W-6229 Kriftel (DE); Siegel, Herbert, Dr., W-6238 Hofheim (DE); Simon, Ulrich, Dr., W-6500 Mainz-Lerchenberg (DE); Thamm, Horst Dieter, Dr., W-6336 Eschborn (DE)

(56) Entgegenhaltungen:
- EP-A- 0 055 814
- EP-A- 0 366 072

## Beschreibung

Die Erfindung betrifft ein verfahren zur Phlegmatisierung von 1,2-Naphthochinon-2-diazid-sulfonsäureestern. Die phlegmatisierten 1,2-Naphthochinon-2-diazid-sulfonsäureester werden insbesondere zu strahlungsempfindlichen Beschichtungslösungen für Halbleitersubstrate oder Druckplattenträgermaterialien verarbeitet.

Strahlungsempfindliche Gemische, die beispielsweise als Flüssigresists bei der Herstellung von Halbleiterbauelementen oder als lichtempfindliche Beschichtung bei der Herstellung von Druckplatten verwendet werden, enthalten in der Regel mindestens ein in wäßrig-alkalischen Lösungen lösliches polymeres Bindemittel und mindestens eine lichtempfindliche Verbindung, die die Löslichkeit des Bindemittels in wäßrig-alkalischen Lösungen herabsetzt. Die lichtempfindliche Verbindung ist bevorzugt ein Ester der gegebenenfalls substituierten 1,2-Naphthochinon-2-diazid-4- oder -5-sulfonsäure mit einem ein- oder mehrwertigen Hydroxyaromaten. Solche Aromaten mit mindestens einer Hydroxygruppe sind beispielsweise Polyhydroxybenzophenone, Polyhydroxydinaphthylmethane, Polyhydroxydiphenylmethane oder 4-(1-Methyl-1-phenyl-ethyl)-phenol (DE-A 26 26 473 = GB-A 1 555 233, DE-A 26 41 099 = US-A 4 163 672).

In der Praxis bevorzugt wegen ihrer relativ hohen Lichtempfindlichkeit und ihrer guten Entwickler-Resistenz sind die mehrfach veresterten Polyhydroxyaromaten. Diese Verbindungen mit ihrem relativ hohen Gehalt an Diazogruppen neigen jedoch zu Explosionen. Ihre Herstellung, Lagerung und Transport dürfen daher nur unter besonderen Vorsichtsmaßnahmen und in kleinen Portionen erfolgen. Die Verarbeitung sollte möglichst umgehend und am selben Ort erfolgen.

Der steigende Verbrauch an Druckplatten und Fotoresists sowie die steigenden Qualitätsanforderungen an diese Produkte machen eine Fertigung an verschiedenen Orten sinnvoll, die nach Möglichkeit weiterhin zentral von einem Produktionsstandort mit lichtempfindlichen Komponenten versorgt werden sollen.

Es sind bereits verschiedene Methoden zur Verminderung der Explosionsgefahr der lichtempfindlichen 1,2-Naphthochinon-2-diazid-sulfonsäureester beschrieben.

Zunächst wurden als lichtempfindliche Substanzen wasserunlösliche Kondensationsprodukte aus 1,2-Naphthochinon-2-diazid-sulfonsäuren und alkalilöslichen Phenol-Formaldehyd-Harzen verwendet (DE-C 865 860 = GB-A 711 626). Analog sind auch Kondensationsprodukte bekannt, die mit Alkylphenol-Formaldehyd-Harzen oder Bisphenol-Formaldehyd-Harzen erhältlich sind (DE-A 30 09 873 = US-A 4 308 368 bzw. DE-A 21 46 166 = GB-A 1 330 932). Zur Herstellung lichtempfindlicher Diazoschichten wurden auch Polyalkylenglykol-chinondiazide, die z.B. durch Umsetzung eines Polyalkylenglykols mit einem Chinondiazid-sulfonyl-chlorid erhältlich sind, eingesetzt (DE-A 20 44 868 = US-A 3 647 443). Die in diesen Schriften genannten Verbindungen erfüllen jedoch nicht mehr die heutigen Anforderungen.

In der EP-A 0 055 814 (≈ US-A 4 424 270 und 4 555 469) wurde bereits die Möglichkeit diskutiert, das Explosionsrisiko der ortho-Chinondiazide durch Abmischen mit Harzen, z.B. den üblicherweise zugesetzten Phenolharzen, oder mit anderen polymeren Verbindungen zu vermindern. Dieser Weg wurde jedoch nicht weiterverfolgt, da es schwierig und gefährlich ist, die in reiner Form isolierten ortho-Chinon-diazide mit den Harzen homogen zu vermischen. Gemäß der genannten EP-A wurde das Problem schließlich dadurch gelöst, daß als lichtempfindliche Komponente in dem lichtempfindlichen Gemisch Ester dienen, die durch Umsetzung eines Naphthochinondiazid-sulfonylhalogenids mit einem Gemisch aus einer niedermolekularen Verbindung, die mindestens eine phenolische Hydroxygruppe enthält, und einer polymeren Verbindung, die mindestens eine solche Hydroxygruppe je wiederkehrende Einheit enthält, hergestellt werden. Die Menge an 1,2-Naphthochinon-2-diazid-sulfonylhalogenid ist dabei größer als die Menge, die zur vollständigen Veresterung der niedermolekularen Phenolkomponente erforderlich ist, so daß auch ein Teil der phenolischen Hydroxygruppen der polymeren Verbindung verestert wird. Von dem so hergestellten Estergemisch geht bereits eine erheblich verminderte Explosionsgefahr aus, es hat jedoch den Nachteil, daß es nur eingeschränkt verwendbar ist.

Eine weitere Möglichkeit, die Gefährlichkeit der Naphthochinonsulfonsäureester zu reduzieren, ist in der EP-A 0 125 587 beschrieben. Gemäß dieser Schrift werden Ester aus 1,2-Naphthochinon-2-diazid-4- oder -5-sulfonsäure und niedermolekularen, phenolische Hydroxygruppen tragenden Verbindungen phlegmatisiert, indem sie durch Fällung aus ihren Lösungen in Gegenwart von unlöslichen, pulverigen, anorganischen Adsorbentien abgeschieden werden. Als Adsorbentien eignen sich besonders Tone, Bleicherde, Aluminiumoxid, Kieselgur, Kieselgel oder ähnliche Mineralien. Ein ähnliches Verfahren zur Phlegmatisierung ist in der EP-A 0 366 072 (= US-A 5 075 193) offenbart. Die 1,2-Naphthochinon-2-diazid-sulfonsäure-ester werden hier jedoch auf mikrokristalline Cellulose abgeschieden.

Das Verschneiden mit Phlegmatisierungsmitteln, die sowohl in Wasser als auch in den bei der Weiterverarbeitung üblicherweise verwendeten organischen Lösemitteln unlöslich sind, hat zwei schwerwiegende Nachteile. Zum einen besteht die Gefahr der Verunreinigung der lichtempfindlichen Komponente durch das Verschnittmittel. Bei einer Verwendung der phlegmatisierten Chinondiazid-Komponente in einem Photoresistgemisch steht dabei die Kontamination durch Metallionen im Vordergrund. Zum anderen müssen die Verschnittmittel aus dem gebrauchsfertigen lichtempfindlichen Gemisch bzw. aus der lichtempfindlichen Beschichtungslösung wieder entfernt sein. Dies wird gewöhnlich durch Filtration erreicht. Bei der Filtration geht jedoch ein Teil des lichtempfindlichen Gemisches verloren, gleichzeitig entstehen mit organischen Verbindungen verunreinigte Rückstände, die fachgerecht beseitigt werden müssen.

Es bestand daher die Aufgabe, ein Verfahren zur Phlegmatisierung zu finden, die die oben genannten Nachteile nicht mehr aufweist.

Gelöst wird die Aufgabe dadurch, daß das Reaktionsgemisch, das bei der Herstellung der 1,2-Naphthochinon-2-diazid-sulfonsäureester nach beendeter Reaktion anfällt, mit einem organischen polymeren Bindemittel vermischt und anschließend das phlegmatisierte Gemisch aus 1,2-Naphthochinon-2-diazid-sulfonsäureester und Bindemittel ausgefällt wird.

Gegenstand der vorliegenden Anmeldung ist daher ein Verfahren zur Phlegmatisierung von 1,2-Naphthochinon-2-diazid-sulfonsäureestern, das dadurch gekennzeichnet ist, daß das aus der Herstellung der 1,2-Naphthochinon-2-diazid-sulfonsäureester hervorgehende Reaktionsgemisch mit einem Phenolharz als Phlegmatisierungsmittel versetzt und anschließend eine phlegmatisierte Mischung, bestehend im wesentlichen aus 1,2-Naphthochinon-2-diazid-sulfonsäureester und Phenolharz, aus dem Gemisch ausgefällt wird.

Die 1,2-Naphthochinon-2-diazid-sulfonsäureester sind insbesondere gebildet aus gegebenenfalls substituierten 1,2-Naphthochinon-2-diazid-4- und/oder -5-sulfonsäuren und einer niedermolekularen, ein- oder mehrwertigen, aromatischen Hydroxyverbindung. Besonders geeignete aromatische Hydroxyverbindungen sind Polyhydroxybenzophenone, wie 2,3,4-Trihydroxy-benzophenon und 2,3,4,4'-Tetrahydroxy-benzophenon, Polyhydroxydiphenylmethane, Polyhydroxydinaphthylmethane, wie 2,2'-Dihydroxy-1,1'-dinaphthylmethan, und 4-(1-Methyl-1-phenyl-ethyl)-phenol.

Von den substituierten 1,2-Naphthochinon-2-diazid-sulfonsäuren sind insbesondere die 7-Alkoxy-1,2-naphthochinon-2-diazid-4- und -5-sulfonsäuren hervorzuheben. Die 7-Alkoxygruppe ist ganz besonders bevorzugt eine Methoxygruppe.

Der Phenolharzanteil beträgt vorteilhaft zwischen 10 und 90 Gew.-%, bevorzugt zwischen 20 und 60 Gew.-%, jeweils bezogen auf das Gesamtgewicht der Mischung.

Bevorzugt ist das polymere Bindemittel identisch mit den üblicherweise in lichtempfindlichen Gemischen verwendeten Bindemitteln, so daß es nicht mehr abgetrennt werden muß, wenn solche Gemische hergestellt werden sollen. Solche Bindemittel sind insbesondere Phenolharze, wie Phenol/Formaldehyd- und Kresol/Formaldehyd-Harze, Kondensationsprodukte aus Hydroxybenzolen, wie Pyrogallol, und Aceton oder aus Bisphenol und Formaldehyd. Da der Bindemittelanteil in den für die Druckplattenherstellung verwendeteten lichtempfindlichen Gemischen gewöhnlich höher ist als der Anteil der lichtempfindlichen (Diazo-)Komponente, wird zu dem erfindungsgemäß hergestellten Gemisch meist noch weiteres Bindemittel im Verlauf der Weiterverarbeitung zugesetzt.

Die nach dem erfindungsgemäßen Verfahren hergestellten phlegmatisierten Gemische fallen nicht unter das Sprengstoffgesetz der Bundesrepublik Deutschland.

Bei Verwendung der erfindungsgemäß phlegmatisierten Gemische ist zudem keine Änderung der Rezeptur notwendig, da das zur Phlegmatisierung verwendete Bindemittel selbst nicht mit 1,2-Naphthochinon-2-diazid-sulfonsäure verestert ist.

Die polymeren Phlegmatisierungsmittel werden zu dem Reaktionsgemisch, das die 1,2-Naphthochinon-2-diazid-sulfonsäureester enthält, entweder in fester Form oder gelöst in einem geeigneten Lösemittel zugegeben. Aus dieser Mischung wird die phlegmatisierte Mischung, bestehend im wesentlichen aus den genannten Estern und den polymeren Bindemitteln, ausgefällt, beispielsweise indem man Wasser oder verdünnte Säure zugibt oder die Mischung in Wasser oder verdünnte Säure einlaufen läßt.

Als Säuren sind Mineralsäuren, vorzugsweise Salzsäure und Schwefelsäure, organische Säuren, wie Oxal- und Malonsäure, aber auch andere Säuren einsetzbar. Die Säuren sind im allgemeinen weniger als 10 gew-%ig. Das in fester Form anfallende phlegmatisierte Gemisch kann nach üblichen Verfahren, wie Filtration, isoliert und anschließend getrocknet werden.

Das erfindungsgemäße Verfahren hat den Vorteil, daß zu keinem Zeitpunkt die reinen Diazoverbindungen isoliert sein müssen. Die bevorzugten Verschnittmittel sind zudem als Bindemittel in lichtempfindlichen Gemischen geeignet.

Für Photoresists eignen sich besonders Gemische, die mit metallionenarmen polymeren Bindemitteln phlegmatisiert sind.

Wie bereits weiter oben angedeutet, eignen sich die erfindungsgemäß phlegmatisierten Gemische in erster Linie zur Herstellung von Beschichtungslösungen für Halbleitersubstrate oder Druckplattenträgermaterialien. Die Herstellung dieser Lösungen erfolgt in an sich bekannter Weise durch Lösen der Komponenten in einem geeigneten Lösemittel. Die Beschichtungslösung kann noch zusätzliche Bestandteile, wie Farbstoffe, Pigmente und photolytische Säurebildner enthalten. Vor der Verwendung wird die Lösung üblicherweise noch einer Feinfiltration unterzogen.

In den folgenden Beispielen steht Gt für Gewichtsteile.

### Beispiel 1:

1,95 Gt 1,2-Naphthochinon-2-diazid-5-sulfonylchlorid und 1,25 Gt 2,2'-Dihydroxy-1,1'-dinaphthylmethan werden in einer Mischung aus 46,8 Gt Aceton und 2 Gt Wasser mit 1 Gt Triethylamin umgesetzt. Nach Beendigung der Umsetzung wird eine Lösung aus 1,25 Gt eines Kresol-Formaldehyd-Novolaks und 3,44 Gt Aceton zugegeben und gut gemischt. Diese Mischung wird innerhalb von 30 Minuten in 62 Gt verdünnte Schwefelsäure (1 %) eingerührt, nach 20 Minuten werden weitere 21 Gt Wasser zugegeben. Anschließend wird die ausgeschiedene Mischung abgesaugt, gewaschen und getrocknet. Die Ausbeute beträgt 4,3 Gt. Der Anteil an Kresol-Formaldehyd-Novolak in dem phlegmatisierten Gemisch beträgt ca. 30 Gew.- %.

### Beispiel 2:

1 Gt 2,3,4-Trihydroxy-benzophenon und 1,9 Gt 1,2-Naphthochinon-2-diazid-5-sulfonylchlorid werden in 9,4 Gt Aceton und 1,6 Gt Wasser mit 1,4 Gt Triethylamin umgesetzt. Zur Beendigung der Reaktion werden 0,6 Gt einer 47%igen Schwefelsäure zugesetzt. Zu dieser Mischung wird eine Lösung aus 1,1 Gt eines Kresol-Formaldehyd-Novolaks in 2,6 Gt Aceton gegeben. Die resultierende Mischung wird in ca. 15 Minuten in 92 Gt Wasser eingerührt. Nach zehnminütigem Rühren läßt man die ausgefallene Suspension 20 Minuten absitzen, trennt die überstehende Lösung ab und rührt nach Zugabe von 42 Gt Wasser nochmals auf. Die Suspension wird abgesaugt, gewaschen und getrocknet. Man erhält 3,1 Gt eines phlegmatisierten Gemisches, das etwa 35 Gew.-% Kresol-Formaldehyd-Harz enthält.

## Patentansprüche

1. Verfahren zur Phlegmatisierung von 1,2-Naphthochinon-2-diazid-sulfonsäureestern, das dadurch gekennzeichnet ist, daß das aus der Herstellung der 1,2-Naphthochinon-2-diazid-sulfonsäureester hervorgehende Reaktionsgemisch mit einem Phenolharz als Phlegmatisierungsmittel versetzt und anschließend eine phlegmatisierte Mischung, bestehend im wesentlichen aus 1,2-Naphthochinon-2-diazid-sulfonsäureester und Phenolharz, aus dem Gemisch ausgefällt wird.

2. Verfahren gemäß Anspruch 1, dadurch gekennzeichnet, daß der Anteil an Phenolharz zwischen 10 und 90 Gew.-%, bevorzugt zwischen 20 und 60 Gew.-%, liegt, jeweils bezogen auf das Gesamtgewicht der Mischung.

3. Verfahren gemäß Anspruch 1 oder 2, dadurch gekennzeichnet, daß die 1,2-Naphthochinon-2-diazid-sulfonsäureester aus gegebenenfalls substituierten 1,2-Naphthochinon-2-diazid-4- und/oder -5-sulfonsäuren und einer niedermolekularen, ein- oder mehrwertigen, aromatischen Hydroxyverbindung gebildet sind.

4. Verfahren gemäß Anspruch 3, dadurch gekennzeichnet, daß die aromatische Hydroxyverbindung ein Polyhydroxybenzophenon, Polyhydroxydiphenylmethan, Polyhydroxydinaphthylmethan oder 4-(1-Methyl-1-phenylethyl)-phenol ist.

5. Verfahren gemäß einem oder mehreren der Anprüche 1 bis 4, dadurch gekennzeichnet, daß das Phenolharz ein Phenol/Formaldehyd- oder Kresol/Formaldehyd-Harz oder ein Kondensationsprodukt aus einem Hydroxybenzol und Aceton oder aus Bisphenol und Formaldehyd ist.

6. Verfahren gemäß einem oder mehreren der Ansprüche 1 bis 5, daduch gekennzeichnet, daß das phlegmatisierte Gemisch durch Zugabe von oder Eintragen in Wasser oder verdünnter Säure ausgefällt, isoliert, gewaschen und getrocknet wird.

## Claims

1. Process for desensitising 1,2-naphthoquinone-2-diazidesulphonic acid esters which is characterised in that the reaction mixture obtained from the production of the 1,2-naphthoquinone-2-diazidesulphonic acid esters is combined with a phenolic resin as a desensitising agent and a desensitised mixture substantially consisting of 1,2-naphthoquinone-2-diazidesulphonic acid esters and phenolic resin is then precipitated from the mixture.

2. Process according to claim 1, characterised in that the proportion of phenolic resin is between 10 and 90 wt.%, preferably between 20 and 60 wt.%, in each case relative to the total weight of the mixture.

3. Process according to claim 1 or 2, characterised in that the 1,2-naphthoquinone-2-diazidesulphonic acid esters are formed from optionally substituted 1,2-naphthoquinone-2-diazide-4- and/or -5-sulphonic acids and a low molecular weight, mono- or polyhydric, aromatic hydroxy compound.

4. Process according to claim 3, characterised in that the aromatic hydroxy compound is a polyhydroxybenzophenone, polyhydroxydiphenylmethane, polyhydroxydinaphthylmethane or 4-(1-methyl-1-phenylethyl)phenol.

5. Process according to one or more of claims 1 to 4, characterised in that the phenolic resin is a phenol/formaldehyde or cresol/formaldehyde resin or a condensation product prepared from a hydroxybenzene and acetone or from bisphenol and formaldehyde.

6. Process according to one or more of claims 1 to 5, characterised in that the desensitised mixture is precipitated by addition of or introduction into water or dilute acid, isolated, washed and dried.

## Revendications

1. Procédé de stabilisation d'esters d'acide 1,2-naphtoquinone-2-diazide-sulfonique qui est caractérisé en ce qu'on ajoute à un mélange réactionnel provenant de la préparation d'esters 1,2-naphtoquinone-2-diazide-sulfoniques une résine phénolique comme stabilisant et ensuite on précipite un mélange stabilisé, constitué essentiellement d'esters 1,2-naphtoquinone-2-diazide-sulfoniques et de résine phénolique, à partir du mélange.

2. Procédé selon la revendication 1, caractérisé en ce que la teneur en résine phénolique est comprise entre 10 et 90 % en poids, de préférence entre 20 et 60 % en poids, par rapport respectivement au poids global du mélange.

3. Procédé selon la revendication 1 ou 2, caractérisé en ce que les esters d'acide 1,2-naphtoquinone-2-diazide-sulfonique sont formés d'esters des acides 1,2-naphtoquinone-2-diazide-4- et/ou -5-sulfoniques et d'un composé hydroxy aromatique mono ou polyfonctionnel de faible poids moléculaire.

4. Procédé selon la revendication 3, caractérisé en ce que le composé hydroxy aromatique est une polyhydroxybenzophénone, un polyhydroxydiphénylméthane, un polyhydroxydinaphtylméthane ou un 4-(1-méthyl-1-phényléthyl)-phénol.

5. Procédé selon l'une ou plusieurs des revendications 1 à 4, caractérisé en ce que la résine phénolique est une résine phénol/formaldéhyde ou crésol/formaldéhyde ou un produit de condensation d'un hydroxybenzène et d'acétone ou de bisphénol et de formaldéhyde.

6. Procédé selon l'une ou plusieurs des revendications 1 à 5, caractérisé en ce que le mélange stabilisé est précipité par addition de ou par incorporation dans l'eau ou de l'acide dilué puis isolé, lavé et séché.
